# EUROPEAN PATENT SPECIFICATION

(11) **EP 1 421 989 B1**
(45) Date of publication and mention of the grant of the patent: **14.06.2006**
(21) Application number: 03257304.0
(22) Date of filing: 19.11.2003
(51) Int. Cl.: B01J 13/10, A01N 25/28

(54) **Microcapsule and production method thereof**
Mikrokapsel sowie Herstellungsverfahren
Microcapsule et procédé de production

(30) Priority: 20.11.2002 JP 2002336123
(43) Date of publication of application: 26.05.2004
(73) Proprietor: Shin-Etsu Chemical Co., Ltd., Chiyoda-ku, Tokyo (JP)
(72) Inventor: Fukasawa, Miyuki, c/o Shin-Etsu Chemical Co., Ltd., Naka Kubiki-gun Niigata-ken (JP); Hayakawa, Kazuhisa, Shin-Etsu Chemical Co., Ltd., Naka Kubiki-gun Niigata-ken (JP)
(74) Representative: Goddard, David John

(56) References cited:
- EP-A- 0 077 956
- US-A- 3 909 444
- US-A- 5 543 162
- PATENT ABSTRACTS OF JAPAN vol. 008, no. 015 (C-206), 21 January 1984 (1984-01-21) & JP 58 183601 A (SHINETSU KAGAKU KOGYO KK), 26 October 1983 (1983-10-26)
- DATABASE WPI Section Ch, Week 200017 Derwent Publications Ltd., London, GB; Class A18, AN 2000-189343 XP002270849 & JP 2000 033259 A (SHIONOGI & CO LTD), 2 February 2000 (2000-02-02)
- PATENT ABSTRACTS OF JAPAN vol. 2003, no. 07, 3 July 2003 (2003-07-03) & JP 2003 070881 A (SHIN ETSU CHEM CO LTD), 11 March 2003 (2003-03-11)

## Description

### Background of the Invention

### 1. Field of the Invention

The present invention relates to a microcapsule comprising an enteric anionic cellulose capsule derivative and gum arabic and to a production method thereof.

### 2. Description of the Related Art

Heretofore, microencapsulation has served such purposes as protecting core material from an external environment, controlling core material emission, and solidifying the surface of liquid. Microencapsulation has been applied in a wide range of fields, including thermal paper, pharmaceutical and food products, as well as cosmetic products, while assorted improvements and proposals have been made to the composition of the microcapsule shell material and its production method.

Gelatin, natural polymers such as cellulose derivatives, and synthetic polymers such as polyamides have been used for microcapsule shells. Currently, gelatin is most commonly used as a substrate material. However, because of recent deepening concern over human infection with transmissible encephalopathy from cattle, cattle being a source of gelatin, there have been growing demands for a microcapsule substrate other than gelatin. Therein, applications of hydroxypropyl methylcellulose phthalate or hydroxypropyl methylcellulose acetate succinate, which are cellulose derivatives of pharmaceutical additives, are particularly effective in the field of pharmaceutical products and the like because, as shown by their pH dependent solubility, they tend to be primarily used as coating agents for controlling drug release.

Previously, when using anionic polymers for shell material, a method for producing microcapsules by coacervate phase separation after adding a coacervating agent of an acid, a base or a salt or the like has been disclosed.

For example, Japanese Provisional Patent Publication No. 63-287543 proposes a method for producing a microcapsule comprising one or more selected from the group consisting of polyvinyl acetal diethylaminoacetate, hydroxypropyl methylcellulose phthalate, and hydroxypropyl methylcellulose acetate phthalate and the like as shell material, wherein the shell material substance is either dissolved in an organic solvent such as ethanol and has an aqueous solution added to it, or made to coacervate (phase separate) by adding an acid or a base.

In addition, from Japanese Patent Provisional Publication No. 2000-33259, a method for producing a microcapsule is known wherein an aqueous solution of hydroxypropyl methylcellulose phthalate or hydroxypropyl methylcellulose acetate succinate is mixed with an aqueous solution of copolymer of methacrylic acid in a same charge state and at an alkaline pH, and then a coacervating agent such as sodium chloride is added thereto for coacervation.

However, with the former method an organic solvent is used in processing, which creates difficulties with residual organic solvent when recovering a capsule and with operational safety and the like. Further, a shell produced by this method is obtained by a single-phase (simple) coacervation of an anionic polymer.

On the other hand, the latter method produces a microcapsule by mixing two or more polymer solutions with the same electric charge, then adding a coacervating agent of a salt such as sodium chloride for coacervating.

With these coacervations, because the anion part is a polymer and the cation part is a monomer of acid or the like, there tends to be a lack of control, such that the whole body of a capsule may harden during the production of the capsule because of the acid completely permeating into the core material, the polymer density may weaken from the shell becoming thinner, or the produced capsule will have a large shrinkage and thus lacks stability.

US patent 5,543,162 describes a method of preparing insoluble polymeric capsules comprising first mixing charged polysaccharide derivative oligomers with a gellable ionic polymer such as gum arabic and then the substances to be encapsulated. This method uses low molecular weight oligomers derived from degradation of substituted long chain polysaccharides. However, the capsules so produced are intended for applications where their contents are released by pressure or heat, and are not suitable for enteric encapsulation for medicinal use and pH-dependent release.

EP-A-0077956 discloses microcapsules with the shell consisting of ethylcellulose and an enteric cellulose dervative, whereby the core may contain a cross-linked gum arabic.

### Summary of the Invention

It is an object of the present invention to provide a microcapsule which can be produced using a low toxicity polymer substrate without using gelatin or an organic solvent which are seen as having a problem with safety. It is also an object of the present invention to provide a production method for such a microcapsule.

The present inventors have, as a result of earnest research into the above problems, discovered that an oil-based material which is immiscible with water is suspended in an aqueous solution of gum arabic as a core material, then mixed with an aqueous alkaline solution of an enteric anionic cellulose derivative so that complex coacervation occurs between the anionic cellulose derivative and the gum arabic which has adsorbed on the oil-based core material and has been suspended. Consequently, a microcapsule having a shell material which comprises not one but both polymers is obtained. Further, according to the present invention, a safe microcapsule can be prepared since gelatin, which is seen as having a problem with safety, is not used, and neither is an organic solvent used in the reaction system.

The present invention was accomplished by stabilizing a suspension of a core material with gum arabic as a protective colloid, then mixing with an aqueous solution of an enteric anionic cellulose derivative to form a microcapsule shell material of a complex of the gum arabic and the enteric anionic cellulose derivative for encapsulating.

The present invention, specifically, provides a microcapsule comprising an oil-based core material which is immiscible with water; and a shell material which comprises an aqueous solution of gum arabic and an enteric anionic cellulose derivative. In addition, the present invention provides a method for producing a microcapsule comprising steps of suspending an oil-based core material which is immiscible with water in an aqueous solution of gum arabic, then adding an aqueous alkaline solution of an enteric anionic cellulose derivative.

According to the present invention, a microcapsule with an oil-based core material can be produced, without using a substrate which has questions about its safety such as gelatin or an organic solvent, from a complex coacervate made using low-toxicity gum arabic and an aqueous alkaline solution of an enteric anionic cellulose derivative. In addition, the obtained microcapsule, when used as a pharmaceutical product, a food product or a pesticide, is non-toxic, is non-soluble in artificial gastric juice (Japanese Pharmacopoeia, First Liquid: Chloride buffer having a pH value of 1.2), and is degraded by artificial intestinal juice (Japanese Pharmacopoeia, Second Liquid: Phosphate buffer having a pH value of 6.8), thus showing pH solubility dependence. Depending on the microcapsule base, the microcapsule may be effective as a controlled-release base since a release time for the core material can be controlled.

### Detailed Description of the Preferred Embodiments

An enteric anionic cellulose derivative of the present invention is, among the three elution-test test liquids shown in the Japanese Pharmacopoeia, an anionic cellulose derivative which is soluble in Japanese Pharmacopoeia Second Liquid (approximately pH 6.8) which corresponds to artificial intestinal juice, and which also is non-soluble in Japanese Pharmacopoeia First Liquid (approximately pH 1.2) which corresponds to other test solutions such as water or artificial gastric juice. Specifically, an aqueous solution of the anionic cellulose derivative may be prepared by dissolving into an aqueous alkaline solution one or more selected from the group consisting of hydroxypropyl methylcellulose phthalate (HPMCP), hydroxypropyl methylcellulose acetate succinate (HPMCAS), and hydroxypropyl methylcellulose acetate phthalate and the like, preferably hydroxypropyl methylcellulose phthalate and/or hydroxypropyl methyl acetate succinate.

Although the molecular weight of the enteric anionic cellulose derivative is not particularly limited, the amount of hydroxypropyl group substitution is preferably 4.0 to 23.0% by weight, in accordance with the measurement method in the Japanese Pharmacopoeia.

An aqueous solution of a base selected from the group consisting of sodium hydroxide, potassium hydroxide, ammonia, ammonium carbonate, and meglumine may be used for the aqueous alkaline solution to be used.

The concentration of the aqueous alkaline solution, for example, if dissolving a cellulose derivative (HPMCP, HPMCAS), for a sodium hydroxide aqueous solution, may be preferably 0.01 to 1 N. The concentration of the substituted cellulose derivative in the whole solution may be preferably 0.1 to 20% by weight. In addition, it may be preferable if the pH value of the prepared solution is made close to the pH value around the dissociation point of an enteric anionic cellulose derivative. Specifically, a pH of 4.5 to 7.0 may be preferable, further preferable may be a pH of 5.0 to 6.5 which is close to the dissociation point of a cellulose derivative. If the pH is less than 4.5, the cellulose derivative may not dissolve. If the pH is more than 7.0, excess alkali may preferentially consume a later-added cross-linking polymer, whereby the microcapsule shell may not generate. It should be noted that a dissociation point of an enteric anionic cellulose derivative is, for example, for hydroxypropyl methylcellulose phthalate, the pH of a neutralized solution wherein a carboxyl group of carboxy benzoyl is ionized in a neutralization titration to dissolve the hydroxypropyl methylcellulose. In addition, for hydroxypropyl methylcellulose acetate succinate, a dissociation point is the pH of a neutralized solution wherein carboxyl groups of succinate and acetyl are ionized in a neutralization titration to dissolve the hydroxypropyl methylcellulose acetate succinate. The pH of the dissociation point for hydropropyl methylcellulose phthalate or hydroxypropyl methylcellulose acetate succinate is close to a pH of 5.0 to 7.0.

The gum arabic used in the present invention may be a viscous sap of an acacia of the Leguminosae family. Its molecular structure is at present uncertain. According to "Shokuhin Tatorui (Food Polysaccharides)", pages 76-84, first published on November 25, 2001, by Saiwai Shobo), its component sugars are 36% by weight of D-glucose, 31 % by weight of L-arabinose, 13% by weight of L-rhamnose, and 18% by weight of D-glucuronic acid, and in addition 2% by weight of protein. The average molecular mass is reported as being between 200,000 to 580,000.

Examples of gum arabic may include gum arabic as given in the Japanese Pharmacopoeia and/or the (Japanese) Food Additives Regulations.

Gum arabic can be extracted from 500 types of acacia trees. Among those, Acacia senegal and Acacia seyal which can be mainly found in the Sudan are well-known. However, a microcapsule of the present invention may use gum arabic taken from any tree as long as formation of the microcapsule is not hindered.

A concentration of the aqueous gum arabic solution may be preferably from 1 to 50% by weight. This is because under 1%, microcapsule shell strength may weaken, and above 50% the solution's consistency may become thick, causing insufficient dispersion of the core material.

The usage ratio of an aqueous alkaline solution of the enteric anionic cellulose derivative to gum arabic has no particular limit as long as the ratio is such that a microcapsule may be produced, but preferably is provided in the preparation of a capsule in a ratio from (90:10) to (50:50) (ratio by weight).

A capsule may be prepared by suspending an oil-based core material in an aqueous solution of preferably 1 to 50% by weight of gum arabic, and then adding an aqueous solution of an alkaline anionic cellulose derivative. Even if this order is reversed a sufficiently strong stabilized microcapsule can be prepared.

As for the oil-based core material that is immiscible with water which is contained within the capsule may include, but not particularly limited to, a pharmaceutical product, a food product, feed, perfume and a pesticide. Fat-soluble vitamins such as vitamin A, D, E or K, water-insoluble or sparingly water-soluble drugs such as nifedipine, and pheromones and the like can be given as specific examples. In particular, since hydroxypropyl methylcellulose phthalate, hydroxypropyl methylcellulose acetate succinate, and chitosan are non-toxic when used in pharmaceutical products or food products, if these are used as shell material they may be effective for applying in such pharmaceutical or food products.

The oil-based core material which is immiscible with water may preferably be an organic material with a boiling point of 100°C or more, and may be for example, a feed, perfume or pheromone material. As an example of a feed or perfume, soybean oil, sunflower oil, palm oil, corn oil, coconut oil, cotton seed oil, castor oil, peanut oil, essential oils (such as rose, jasmine, orange, and lime), and natural plant or animal glycerides such as soybean fatty acids, animal fat, bacon grease, or lard and fish oil can be given. In addition, as examples of a pheromone material, Z-7-dodecenyl acetate, Z-8-dodecenyl acetate, Z-9-dodecenyl acetate, E, Z-7, 9-dodecadienyl acetate, Z, Z-7, 9-dodecadienyl acetate, E, E-8, 10-dodecadienol, E-4-tridecenyl acetate, Z-9-tetradecenyl acetate, Z-9-tetradecenal, Z-11-tetradecenyl acetate, Z-11-tetradecenal, Z-9-hexadecenal, Z-11-hexadecenal, Z, E-9, 11-tetradecadienyl acetate, Z, E-9, 12-tetradecadienyl acetate, Z-11-hexadecenyl acetate, Z, Z-7, 11-tetradecadienyl acetate, Z, E-7, 11-tetradecadienyl acetate, E, E, Z-4, 6, 10-hexadecatrienyl acetate, E, E-10, 12-hexadecadienal, Z, Z-3, 13-octadecadienyl acetate, E, Z-3, 13-octadecadienyl acetate, Z-7, 8-epoxy-2-methyl-octadecene, Z-13-icosene-10-one, E, E, Z-10, 12, 14-hexadecatrienyl acetate, E, E, Z-10, 12, 14-hexadecatrienal, Z-10-tetradecenyl acetate, E, Z-4, 10-tetradecadienyl acetate, 14-methyl-1-octadecene, (R, Z)-5-(1-octenyl)oxacyclopentane-2-one, (R, Z)-5-(1-decenyl)oxacyclopentane-2-one, and the like can be given.

In addition, in a Japanese Pharmacopoeia First Liquid, a microcapsule obtained from hydroxypropyl methylcellulose phthalate and chitosan, or hydroxypropyl methylcellulose acetate succinate and chitosan, essentially showed no change. However, in a Japanese Pharmacopoeia Second Liquid, pH dependent releasibility was shown by rapid disintegration, and thus it is learnt that such a microcapsule may be effective as a controlled release base. The form of the core material to be used may be any of a solid, semi-solid, liquid or gas.

The method for suspending a core material in an aqueous solution of gum arabic is not particularly limited, and any commonly performed method is acceptable. For example, a stirrer or an emulsifier may be used.

A preferable additive amount for the core material may be 1 to 100 parts by weight per 100 parts by weight of gum arabic.

By suspending an oil-based material, which is immiscible with water, in an aqueous solution of gum arabic, then mixing with an aqueous alkaline solution of an enteric anionic cellulose derivative, complex coacervation occurs between the anionic cellulose derivative and the gum arabic which has adsorbed on the oil-based core material and has been suspended, thereby allowing a microcapsule comprising shell material which comprises both of the polymers to be obtained.

An apparatus used for producing a microcapsule of the present invention may not be particularly limited, and any apparatus which is commonly used in complex coacervation may be acceptable.

A microcapsule form of the present invention may be, preferably, a spherical or a spindle shape with an average diameter of 1 to 10⁴µm. In addition, the form may be any of a form wherein the shell material encloses the core material, or wherein the core material is dispersed within the shell material (beads), or multilayered capsules or the like. The microcapsule form can be controlled by the stirring speed after adding the aqueous solution of the anionic cellulose derivative.

It may be noted that "microcapsule" as mentioned in the present invention is not particularly limited if produced in the same manner as that of the present invention, and may include any of the names such as microsphere, nanocapsule, centicapsule, and seamless capsule and the like.

In a microcapsule of the present invention, material such as polyhydric alcohol or a surfactant may be optionally added for the purpose of obtaining suspension stability of the core material. In order to further strengthen the microcapsule shell, acid treatment may be performed with hydrochloric acid, acetic acid, phosphoric acid, or the like, with a pH of 3 to 5 desirable. Below this pH, due to excess acid only anionic polymers may preferentially be coacervated. If an aqueous solution of methylcellulose is added, capsule size can be controlled.

A capsule obtained by coacervation may be used as is in its dispersed state in water. Alternatively, the capsule obtained are filtered, dried and stored so that the stored capsule will be used in a required amount thereof.

Herein, the present invention will be further specifically explained using examples. However, it should be noted that the present invention is not limited to such examples.

### Example 1

The 40g of hydroxypropyl methylcellulose phthalate (HP-55S: manufactured by Shin-Etsu Chemical Co., Ltd.) was mixed with and then dissolved into 180g of an aqueous 0.5 N sodium hydroxide solution. The 60g of the resulting solution was then mixed with 40g of deionized water to give an anionic polymer solution (pH 5.3). Next, 0.5g of gum arabic (manufactured by Wako Pure Chemical Industries, Ltd.) was added to deionized water to give a 1% by weight aqueous gum arabic solution.

To this aqueous gum arabic solution, 0.2mg of riboflavin phosphate sodium (manufactured by Wako Pure Chemical Industries, Ltd.) was dissolved. Core liquid was made from 20g of the obtained solution, stirred with a magnetic stirrer (300rpm) and gradually allowed to drip into the hydroxypropyl methylcellulose phthalate. After being stirred for some time, the solution was filtered, and allowed to dry to obtain a microcapsule.

### Example 2

The 40g of hydroxypropyl methylcellulose phthalate (HP-55S: manufactured by Shin-Etsu Chemical Co., Ltd.) was mixed and then dissolved into 180g of an aqueous 0.5 N sodium hydroxide solution. The 80g of the resulting solution was then mixed with 20g of deionized water to give an anionic polymer solution (pH 5.3). Next, 0.5g of gum arabic (manufactured by Wako Pure Chemical Industries, Ltd.) was added to deionized water to give a 1% by weight aqueous gum arabic solution. The 10g of vitamin E (manufactured by Eisai Co., Ltd.) was added to 90g of the obtained aqueous gum arabic solution, and suspended using a homogenizer to give a core liquid. The core liquid was stirred with a magnetic stirrer (300rpm) and gradually allowed to drip into the hydroxypropyl methylcellulose phthalate. After being stirred for some time, the solution was filtered, and allowed to dry to obtain a microcapsule.

### Example 3

The 15g of hydroxypropyl methylcellulose acetate succinate (HPMCAS / Shin-Etsu AQOAT: manufactured by Shin-Etsu Chemical Co., Ltd.) was mixed with and then dissolved into 85g of an aqueous 0.2 N sodium hydroxide solution to give an anionic polymer solution (pH 5.5). Except for this replacing the anionic polymer solution of Example 1, a microcapsule was prepared in the same manner as Example 1.

### Example 4

The 15g of hydroxypropyl methylcellulose acetate succinate (HPMCAS / Shin-Etsu AQOAT: manufactured by Shin-Etsu Chemical Co., Ltd.) was mixed with and then dissolved into 85g of an aqueous 0.2 N sodium hydroxide solution to give an anionic polymer solution (pH 5.5). Except for this replacing the anionic polymer solution of Example 2, a microcapsule was prepared in the same manner as Example 2.

### Test Example 1

A microcapsule prepared as in Example 1 was left in artificial gastric juice (Japanese Pharmacopoeia First Liquid; pH 1.2) for 1 hour. Upon observation, the capsule showed no change in appearance. Then, the microcapsule was left in artificial intestinal juice (Japanese Pharmacopoeia First Liquid; pH 6.8) for 1 hour, whereupon the microcapsule shell was seen to have swollen and core material released.

### Test Example 2

A microcapsule prepared as in Example 4 was left in artificial gastric juice (Japanese Pharmacopoeia First Liquid; pH 1.2) for 1 hour. Upon observation, the capsule showed little change in appearance. Then, the microcapsule was left in artificial intestinal juice (Japanese Pharmacopoeia First Liquid; pH 6.8) for 1 hour, whereupon the microcapsule shell was seen to have disintegrated and core material released.

## Claims

1. A microcapsule comprising oil-based core material which is immiscible with water; and shell material which comprises gum arabic and an enteric anionic cellulose derivative.

2. The microcapsule of claim 1, wherein said enteric anionic cellulose derivative is one or more selected from the list consisting of hydroxypropyl methylcellulose phthalate, hydroxypropyl methylcellulose acetate succinate and hydroxypropyl methylcellulose acetate phthalate.

3. The microcapsule of claims 1 or 2, wherein said oil-based core material is an organic compound with a boiling point of 100°C or greater.

4. The microcapsule of any one of claims 1 to 3, wherein said oil-based core material is selected from the group consisting of fat-soluble vitamins, water-insoluble or sparingly water-soluble drugs, and pheromones.

5. A method for producing a microcapsule comprising steps of suspending an oil-based water-immiscible core material in an aqueous solution of gum arabic, and then adding an aqueous solution of an enteric anionic cellulose derivative.

6. The method according to claim 5 wherein said enteric anionic cellulose derivative is one or more selected from the group consisting of hydroxypropyl methylcellulose phthalate, hydroxypropyl methylcellulose acetate succinate and hydroxypropyl methylcellulose acetate phthalate

7. The method according to either of claims 5 or 6 wherein said aqueous solution of an enteric anionic cellulose derivative has a pH in the range 4.5 to 7.0.

8. The method according to claim 7 wherein said aqueous solution of an enteric anionic cellulose derivative has a pH in the range 5.0 to 6.5.

9. The method according to any one of claims 5 to 8, wherein said aqueous solution of an enteric anionic cellulose derivative is dissolved in an alkaline solution.

## Patentansprüche

1. Mikrokapsel, umfassend ein Kernmaterial auf Ölbasis, das mit Wasser nicht mischbar ist, und ein Hüllenmaterial, das Gummiarabikum und ein enterisches, anionisches Cellulosederivat umfaßt.

2. Mikrokapsel nach Anspruch 1, wobei es sich bei dem enterischen, anionischen Cellulosederivat um eines oder mehrere handelt, die aus der Liste ausgewählt sind, welche aus Hydroxypropylmethylcellulosephthalat, Hydroxypropylmethylcelluloseacetatsuccinat und Hydroxypropylmethylcelluloseacetatphthalat besteht.

3. Mikrokapsel nach Anspruch 1 oder Anspruch 2, wobei das Kernmaterial auf Ölbasis eine organische Verbindung mit einem Siedepunkt von 100°C oder mehr ist.

4. Mikrokapsel nach einem der Ansprüche 1 bis 3, wobei das Kernmaterial auf Ölbasis aus der Gruppe ausgewählt ist, die aus fettlöslichen Vitaminen, wasserunlöslichen oder kaum wasserlöslichen Arzneimitteln und Pheromonen besteht.

5. Verfahren zur Herstellung einer Mikrokapsel, das die Schritte des Suspendierens eines nicht mit Wasser mischbaren Kernmaterials auf Ölbasis in einer wäßrigen Lösung von Gummiarabikum und des anschließenden Zugebens einer wäßrigen Lösung eines enterischen, anionischen Cellulosederivates umfaßt.

6. Verfahren nach Anspruch 5, wobei es sich bei dem enterischen, anionischen Cellulosederivat um eines oder mehrere handelt, die aus der Gruppe ausgewählt sind, welche aus Hydroxypropylmethylcellulosephthalat, Hydroxypropylmethylcelluloseacetatsuccinat und Hydroxypropylmethylcelluloseacetatphthalat besteht.

7. Verfahren nach Anspruch 5 oder Anspruch 6, wobei die wäßrige Lösung eines enterischen, anionischen Cellulosederivats einen pH-Wert im Bereich von 4,5 bis 7,0 aufweist.

8. Verfahren nach Anspruch 7, wobei die wäßrige Lösung eines enterischen, anionischen Cellulosederivats einen pH-Wert im Bereich von 5,0 bis 6,5 aufweist.

9. Verfahren nach einem der Ansprüche 5 bis 8, wobei die wäßrige Lösung eines enterischen, anionischen Cellulosederivats in einer alkalischen Lösung gelöst ist.

## Revendications

1. Micro capsule comprenant un matériau noyau à base d'huile, qui n'est pas miscible dans l'eau, et un matériau d'enrobage qui comporte de la gomme arabique et un dérivé de cellulose anionique entérique.

2. Micro capsule selon la revendication 1, **caractérisée en ce que** le dérivé cellulose anionique entérique est un ou plusieurs choisi parmi la liste consistant en hydroxypropyl, méthylcellulose acétate succinate et hydroxypropyl méthylcellulose acétate phthalate.

3. Micro capsule selon l'une des revendications 1 ou 2, **caractérisée en ce que** ledit matériau noyau à base d'huile est un composé organique avec un point d'ébullition de 100 °C ou plus.

4. Micro capsule selon l'une des revendications 1 à 3, **caractérisée en ce que** ledit matériau noyau à base d'huile est choisi parmi le groupe consistant en des vitamines solubles dans des matières grasses, insolubles dans l'eau, des drogues solubles modérément dans l'eau.

5. Procédé pour la production d'une micro capsule comprenant les étapes de mise en suspension d'un matériau de noyau à base d'huile et qui n'est pas miscible dans l'eau, dans une solution aqueuse de gomme arabique, et ensuite ajouter une solution aqueuse d'un dérivé de cellulose anionique entérique.

6. Procédé selon la revendication 5, **caractérisé en ce que** ledit dérivé cellulose anionique entérique est un ou plusieurs choisis parmi le groupe consistant en hydroxypropyl méthylcellulose acétate phthalate, hydroxypropyl méthylcellulose acétate succinate et hydroxypropyl méthylcellulose acétate phthalate.

7. Procédé selon l'une des revendications 5 ou 6, **caractérisé en ce que** ladite solution aqueuse d'un dérivé de cellulose anionique entérique présente un pH dans la gamme de 4,5 à 7,0.

8. Procédé selon la revendication 7, **caractérisé en ce que** ladite solution aqueuse d'un dérivé de cellulose anionique entérique présente un pH dans la gamme de 5,0 à 6,5.

9. Procédé selon l'une des revendications 5 à 8, **caractérisé en ce que** ladite solution aqueuse d'un dérivé de cellulose anionique entérique est dissoute dans une solution alcaline.
